# EUROPEAN PATENT SPECIFICATION

(11) **EP 0 973 519 B1**
(45) Date of publication and mention of the grant of the patent: **14.08.2002**
(21) Application number: 98906851.5
(22) Date of filing: 25.02.1998
(51) Int. Cl.: A61K 31/40, A61K 31/35, A61P 19/08, A61P 19/10

(54) **INCLUSION COMPLEXES IN AQUEOUS SOLUTION**
INKLUSIONSKOMPLEXE IN WAESSRIGER LOESUNG
COMPLEXES D'INSERTION EN SOLUTION AQUEUSE

(30) Priority: 27.02.1997 DK 21697
(43) Date of publication of application: 26.01.2000
(73) Proprietor: NOVO NORDISK A/S, 2880 Bagsvaerd (DK)
(72) Inventor: RASMUSSEN, Stella, Rudkjaer, DK-2100 Copenhagen (DK)
(74) Representative: Kjerrumgaard, Lars
(86) International application number: DK9800073
(87) International publication number: WO9837884

(56) References cited:
- EP-A- 0 672 412
- WO-A-94/20098

## Description

This invention relates to novel inclusion complexes in aqueous solution, and pharmaceutical formulations thereof, which are useful for the treatment of certain medical indications in mammals.

### Background of the Invention

The 3,4-diarylchroman of formula I 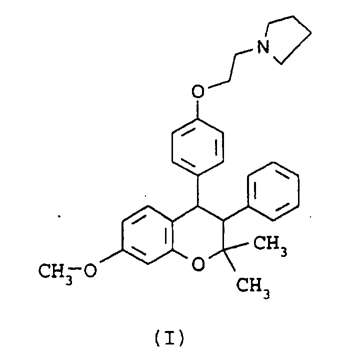 and pharmaceutically acceptable salts thereof are well known in the art, see e.g. U.S. Patent No. 5,280,040. This patent describes the preparation of these compounds, as well as their use in reducing bone loss. The preparation of pharmaceutical compositions thereof is also described. These compounds are also known to be effective in the treatment of a variety of other medical indications e.g. as described in WO 96/21442, WO 96/21443, WO 96/21444, WO 96/22091, WO 96/22092 and WO 96/22093. 3,4-trans-2,2-dimethyl-3-phenyl-4-[4-(2-pyrrolidin-1-yl)ethoxy)phenyl]-7-methoxychroman with the above formula I, also known as centchroman, is a non-steroidal compound known to have antiestrogenic activity. It is in use in India as an oral contraceptive (see, for example, Salman et al., U.S. Patent No. 4,447,622; Singh et al., Acta Endocrinal (Copenh) 126 (1992), 444 - 450; Grubb, Curr Opin Obstet Gynecol 3 (1991), 491 - 495; Sankaran et al., Contraception 9 (1974), 279 - 289; Indian Patent Specification No. 129187). Centchroman has also been investigated as an anti-cancer agent for treatment of advanced breast cancer (Misra et al., Int J Cancer 43 (1989), 781 - 783. Recently, centchroman as a racemate has been found as a potent cholesterol lowering pharmaceutical agent expressed by a significant decrease of the serum concentrations (S.D. Bain et al., J Min Bon Res 9 (1994), S 394). It is preferred to use the compounds of formula I in the trans configuration. The I enantiomeric forms are preferred over racemic mixtures.

The 3,4-diarylchromans are prepared according to known methods, such as those disclosed in U.S. Patent No. 3,340,276 to Carney et al., U.S. Patent No. 3,822,287 to Bolger, and Ray et al., J Med Chem 19 (1976), 276 - 279, the contents of which are incorporated herein by reference. Conversion of the cis isomer to the trans configuration by means of an organometallic base-catalyzed rearrangement is disclosed in U.S. Patent No. 3,822,287. The optically active d- and I-enantiomers may be prepared as disclosed by Salman et al. in U.S. Patent No. 4,447,622 (incorporated herein by reference) by forming an optically active acid salt which is subjected to alkaline hydrolysis to produce the desired enantiomer. The resolvation of (+/-)-3,4-trans-7-methoxy-2,2-dimethyl-3-phenyl-4-{4-[2-(pyrrolidin-1-yl)ethoxy]phenyl}chromane in its optical antipodes is described in U.S. Patent No. 4,447,622 incorporated herein by reference. Example 1 of U.S. Patent No. 4,447,622 describes the preparation of the minus enantiomer, shown by formula II : 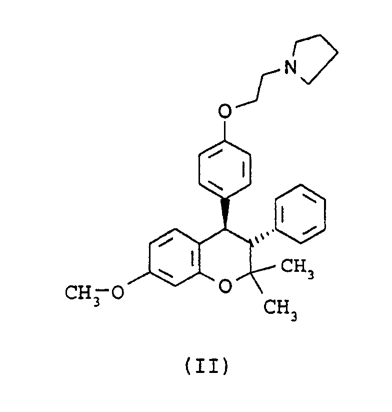 (In this specification, the compound of formula II is referred to as levormeloxifene.) In example 2 of U.S. Patent No. 4,447,622, levormeloxifene is obtained as the free base and the hydrochloride salt. Levormeloxifene, ( - ) - 3R,4R - trans- 7-methoxy-2,2-dimethyl-3-phenyl-4-{4-[2-(pyrrolidin-1-yl)ethoxy]phenyl}chromane, is a particular preferred compound. Levormeloxifene may be used in human and veterinary medicine for the regulation of bone metabolism. It may be used, for example, in the treatment of patients suffering from bone loss due to osteoporosis (including post-menopausal osteoporosis and glucocorticoid-related osteoporosis), Paget's disease, hyperparathyroidism, hypercalcemia of malignancy and other conditions characterized by excessive rates of bone resorption and/or decreased rates of bone formation.

Compounds of formula I, and particularly acid salt forms of such compounds including, for example, hydrogen fumarate, hydrochloride, sulfate, hydrobromide, citrate and the like, generally are poorly water soluble under ambient temperature. Because of this poor water solubility, it presently is necessary to administer these compounds as a suspension in water using a suspending agent such as carboxymethyl cellulose (CMC), polyethylene glycol, and the like. However, the above-mentioned pharmaceutical formulations used with compounds of formula I cannot be utilized in most administration forms.

Particularly, formulations useful for intravenous (IV) administration must be in the form of a solution. The IV administration of a suspension is extremely dangerous because particulate material in suspension can lodge in the microvasculature of a mammal causing life-threatening blockages and embolisms.

Water soluble formulations also are necessary for intranasal and (aerosol) pulmonary administration of pharmaceutical agents because water solubility is necessary for such agents to cross upper and lower respiratory tract membranes. Failure to provide water soluble forms of these agents generally leads to poor drug absorption and/or irritation of the respiratory tract.

Additionally, it is desirable, although less critical, to have water soluble formulations available for other routes of administration. For example, transdermal or liquid formulations for oral administration are desirable because they are more homogenous than other forms of pharmaceutical agents, and therefore, provide better dispersion and absorption in the GI tract. A water soluble formulation of a pharmaceutical agent also provides greater safety and convenience for a patient and the attending physician.

The present invention provides novel inclusion complexes in aqueous solution, pharmaceutical compositions thereof, and methods of using such complexes.

The present invention provides an inclusion complex in aqueous solution comprising a compound of formula I above and a water soluble cyclodextrin.

Also provided by the present invention is a pharmaceutical composition comprising an inclusion complex in aqueous solution as described above, in combination with a pharmaceutically acceptable carrier, diluent or excipient.

One aspect of the present invention provides an inclusion complex in aqueous solution comprising a compound of formula I 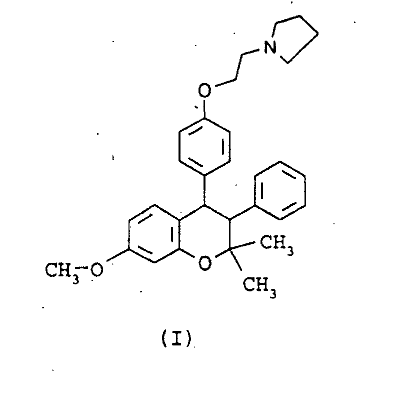 or a pharmaceutically acceptable salt thereof, and a water soluble cyclodextrin.

The compounds used in the methods of this invention form pharmaceutically acceptable salts such as acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycolate, heptanoate, hippurate, lactate, malate, maleate, hydroxymaleate, malonate, mandelate, mesylate, nicotinate, isonicotinate, nitrate, oxalate, phthalate, terephthalate, phosphate, monohydrogenphosphate, dihydrogenphosphate, metaphosphate, pyrophosphate propiolate, propionate, phenylpropionate, salicylate, sebacate, succinate, suberate, sulfate, bisulfate, pyrosulfate, sulfite, bisulfite, sulfonate, benzenesulfonate, p-bromophenylsulfonate, chlorobenzenesulfonate, ethanesulfonate, 2-hydroxyethanesulfonate, methanesulfonate, naphthalene-1-sulfonate, naphthalene-2-sulfonate, p-toluenesulfonate, xylenesulfonate, tartarate, and the like.

In one preferred embodiment of the invention, the formula I compound is (-)-3R,4R-trans-7-methoxy-2,2-dimethyl-3-phenyl-4-{4-[2-(pyrrolidin-1-yl)ethoxy]phenyl}chromane. Especially preferred is the hydrogen fumarate salt form of this compound.

Thus, compounds of formula I represent one of the starting materials of the present invention which, in the presence of the other starting material, a water soluble cyclodextrin, forms inclusion complexes in aqueous solution according to the present invention.

Cyclodextrins are cyclic molecules containing six or more α-D-glycopyranose units linked at the 1,4-positions by α linkages as in amylase. Because of this cyclic arrangement, the molecule is characterized as having neither a reducing end group nor a non-reducing end group. It is, therefore, believed that there is limited free rotation about the glycosidic bonds, and the cyclodextrins exist as conical-shaped molecules with the primary hydroxyls situated at the small end of the cone and the secondary hydroxyls situated at the large opening of the cone. The central cavity, formed by the conformation of the cyclic α-D-glycopyranose units, is lined by hydrogen atoms and oxygen atoms resulting in a relatively lipophilic cavity, but the outer surface is hydrophilic. Cyclodextrins, therefore, have the ability to form complexes with some organic and inorganic molecules.

Numerous cyclodextrins and methods for their preparation have been described. For example, Parmeter (I), *et al.* (U.S. Patent No. 3,453,259) and Gramera, *et al.* (U.S. Patent No. 3,459,731) described electroneutral cyclodextrins. Other derivatives include cyclodextrins with cationic properties [Parmeter (II), U.S. Patent No. 3,453,257], insoluble cross-linked cyclodextrins (Solms, U.S. Patent No. 3,420,788), and cyclodextrins with anionic properties [Parmeter (III), U.S. Patent No. 3,426,011]. Among the cyclodextrin derivatives with anionic properties, carboxylic acids, phosphorous acids, phosphinous acids, phosphonic acids, phosphoric acids, thiophosphonic acids, thiosulphinic acids, and sulfonic acids have been appended to the parent cyclodextrin [see, Parmeter (III), supra.] Furthermore, sulfoalkyl ether cyclodextrin derivatives have been described by Stella, *et al.* (U.S. Patent No. 5,134,127).

Although this broad variety of cyclodextrins is described in the above patents and it is known that cyclodextrins may be useful in preparing pharmaceutical agents for certain pharmaceutical delivery systems, it is also well recognized in the art that cyclodextrins will not assist in preparing such systems with all pharmaceutical agents.

Thus, use of cyclodextrins in the present invention is limited to water soluble cyclodextrins which form aqueous solutions upon the addition of water. The water solubility of cyclodextrins either is known in the art or may be determined via known procedures. Of the water soluble cyclodextrins, use of hydroxyalkyl-β-cyclodextrins (see, e.g., U.S. Patent No. 4,727,064), such as hydroxy-C₁₋₆alkyl-β-cyclodextrins and, particularly, hydroxypropyl-β-cyclodextrin, is preferred for preparing the inclusion complexes of the present invention.

Typically, the inclusion complexes in aqueous solution according to the present invention are prepared by adding water to the desired water soluble, synthesized or commercially available (see, e.g., Janssen Chimica, Geel, Belgium; Sigma Chemical Company, St. Louis, MO; Aldrich Chemical Company, Inc., Milwaukee, WI; Pharmtec, Alachuo, FL; and Lancaster Synthesis Inc., Windham, NH), cyclodextrin and a compound of formula I. The concentration of cyclodextrin is from 1% to 20% and preferably from about 1.5% to about 10%, more preferred from about 2% to about 7%. Cyclodextrin concentrations of about 0.5% or less are not desirable. The concentration of the specific compound of formula I in the final inclusion complex is from 0.1 mg/ml to about 10 mg/ml, preferably from about 0.5 mg/ml to about 5 mg/ml and more preferred from about 0.5 mg/ml to about 2 mg/ml.

The mixture of water, cyclodextrin and a compound of formula I is stirred for 15 to 60 minutes until the solution becomes clear.

The preparation of inclusion complexes in aqueous solution according to the present invention usually is carried out at room temperature.

The pH of these inclusion complexes is slightly acidic to about neutral (from about 4.0 to about 7.0). The pH generally need not be adjusted prior to preparation of pharmaceutical compositions.

Thus, the present invention also provides pharmaceutical compositions comprising an inclusion complex in aqueous solution according to the present invention in combination with a pharmaceutically acceptable carrier, diluent, or excipient. Such pharmaceutical compositions are prepared in a manner well known in the pharmaceutical art, and are administered individually or in combination with other therapeutic agents, preferably via parenteral routes. An especially preferred route is intravenous. Other preferred routes of administration include oral, transdermal , intranasal, and pulmonary.

In making the compositions of the present invention, active ingredient, which comprises at least one inclusion complex in aqueous solution according to the present invention, is usually mixed with the excipient or diluted by an excipient. When an excipient is used as a diluent, it should be a liquid material which acts as a vehicle, carrier, or medium for the active ingredient.

Some examples of suitable excipients include water and syrup, and the formulations can additionally include wetting agents, preserving agents such as methyl- and propylhydroxybenzoates, additional sweetening agents, and flavoring agents.

The compositions are preferably formulated in a unit dosage form with each dosage normally containing from about 1 ml to about 100 ml, more usually from about 20 ml to about 60 ml of the active ingredient in aqueous solution. The term "unit dosage form" refers to physically discrete units suitable as unitary dosages for human subjects and other mammals, each unit containing a predetermined quantity of active material calculated to produce the desired therapeutic effect in association with a suitable pharmaceutial excipient.

The particular dosage of an inclusion complex in aqueous solution according to the present invention required to treat the aforementioned medical indications will depend upon the severity of the disease or condition, its route of administration, and related factors that will be decided by the attending physician. Generally, accepted and effective dosages will be from about 0.1 mg to about 100 mg, and more typically, from about 0.5 mg to about 50 mg. Such dosages will be administered to a mammal in need of such treatment from about once a week to about once a day.

The following formulation examples only are illustrative and are not intended to limit the scope of the present invention in any way.

### Formulation 1

An intraveneous formulation may be prepared as follows:

| | |
|---|---|
| Compound of formula I | 12.5 mg |
| Cyclodextrin | 200 mg |
| Glucose | 506 mg |
| Water for injection q.s.to | 10g |

### Formulation 2

An aerosol solution may be prepared containing the following components:
10 ml of 20% cyclodextrin solution containing
10 mg of a compound of formula I
25% ethanol; and
70% Propellant 22® (chlorodifluoromethane).

### Formulation 3

An oral formulation may be prepared containing the following components

| | |
|---|---|
| Compound of formula I | 25 mg |
| Cyclodextrin | 300 mg |
| Water q.s. to | 10g |

We have found that compounds of formula I would form aqueous solutions when mixed with a water soluble cyclodextrin in water.

We further found that an inclusion complex in aqueous solution according to the present invention resulted in a greater than 7 fold increase in water solubility.

The present invention further provides a method for inhibiting bone loss comprising administering to a mammal, particularly a postmenopausal woman, in need of treatment an effective amount of an inclusion complex in aqueous solution according to the present invention.

The term "inhibit" includes its generally accepted meaning which includes prohibiting, preventing, restraining, and slowing, stopping or reversing. As such, the present method includes both medical therapeutic and/or prophylatic administration, as appropriate.

As used herein the term "mammal" includes humans e.g. men, women and children.

The following example is provided to further illustrate the present invention. It is not intended that the invention be limited in scope by reason of the following example.

### Example 1

Preparation of levormeloxifene-hydroxypropyl-β-cyclodextrin inclusion complex.

A solution containing 20 g Hydroxypropyl-β-cyclodextrin, 1.25 g levormeloxifene, fumarate was prepared by adding 1000 ml of water. The mixture was stirred until it became a clear solution.

## Claims

1. An inclusion complex in aqueous solution comprising a compound of formula I 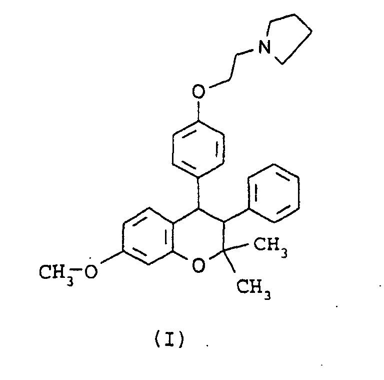 or a pharmaceutically acceptable salt thereof, and a water soluble cyclodextrin.

2. An inclusion complex in aqueous solution according to claim 1 wherein the compound of formula I is (-) - 3R,4R - trans- 7-methoxy-2,2-dimethyl-3-phenyl-4-{4-[2-(pyrrolidin-1-yl)ethoxy]phenyl}chromane or a salt thereof.

3. An inclusion complex according to anyone of the above claims wherein said salt thereof is a hydrogen fumarate salt.

4. An inclusion complex according to anyone of the above claims wherein the concentration of a compound of formula I is from about 0.1 mg/ml to about 10mg/ml, more preferred from about 0.5 mg/ml to about 5 mg/ml.

5. An inclusion complex according to anyone of the above claims wherein the concentration of a cyclodextrin is above 0,5 %, preferably from about 1,5 % to about 10% and more preferred from about 2% to about 7%.

6. An inclusion complex according to anyone of the above claims wherein said water soluble cyclodextrin is selected from hydroxyalkyl-β-cyclodextrins, such as hydroxypropyl-β-cyclodextrin.

7. A pharmaceutical formulation comprising as an active ingredient an inclusion complex as claimed in any one of claims 1 to 6, associated with one or more pharmaceutically acceptable carriers, excipients, or diluents therefor.

8. An inclusion complex as claimed in any one of claims 1 to 6 for use as a bone loss inhibiting agent.

9. The use of an inclusion complex as claimed in any one of claims 1 to 6 for the manufacture of a pharmaceutical formulation for inhibiting bone loss.

10. A process for preparing an inclusion complex of any one of claims 1 to 6, which comprises adding an appropriate amount of a water soluble cyclodextrin and a compound of formula I or a salt hereof, to water, stirring the mixture until the mixture becomes clear.

## Patentansprüche

1. Einschlusskomplex in wässriger Lösung, der eine Verbindung der Formel I 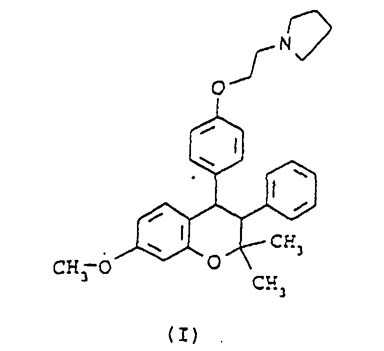 oder ein pharmazeutisch geeignetes Salz davon und ein wasserlösliches Cyclodextrin umfasst.

2. Einschlusskomplex in wässriger Lösung nach Anspruch 1, bei dem die Verbindung der Formel I (-)-3R,4R-trans-7-Methoxy-2,2-dimethyl-3-phenyl-4-{4-[2-(pyrrolidin-1-yl)ethoxy]phenyl}chroman oder ein Salz davon ist.

3. Einschlusskomplex nach einem der obigen Ansprüche, bei dem das Salz davon ein Hydrogenfumaratsalz ist.

4. Einschlusskomplex nach einem der obigen Ansprüche, bei dem die Konzentration einer Verbindung der Formel I von etwa 0,1 mg/ml bis etwa 10 mg/ml ist, besonders bevorzugt von etwa 0,5 mg/ml bis etwa 5 mg/ml.

5. Einschlusskomplex nach einem der obigen Ansprüche, bei dem die Konzentration eines Cyclodextrins oberhalb 0,5 % ist, vorzugsweise von etwa 1,5 % bis etwa 10 % und besonders bevorzugt von etwa 2 % bis etwa 7 %.

6. Einschlusskomplex nach einem der obigen Ansprüche, bei dem das wasserlösliche Cyclodextrin ausgewählt ist aus Hydroxyalkyl-β-cyclodextrinen, wie Hydroxypropyl-β-cyclodextrin.

7. Pharmazeutische Formulierung, die als einen aktiven Bestandteil einen Einschlusskomplex wie in einem der Ansprüche 1 bis 6 beansprucht umfasst, assoziiert mit einem oder mehreren pharmazeutisch dafür geeigneten Trägerstoffen, Hilfsstoffen oder Verdünnungsmittlen.

8. Einschlusskomplex wie beansprucht in einem der Ansprüche 1 bis 6 zur Verwendung als ein Knochenverlust-inhibierendes Mittel.

9. Verwendung eines Einschlusskomplexes wie beansprucht in einem der Ansprüche 1 bis 6 für die Herstellung einer pharmazeutischen Formulierung zum Inhibieren von Knochenverlust.

10. Verfahren zum Herstellen eines Einschlusskomplexes nach einem der Ansprüche 1 bis 6, welches das Zugeben einer geeigneten Menge eines wasserlöslichen Cyclodextrins und einer Verbindung der Formel I oder eines Salzes davon zu Wasser, Rühren der Mischung, bis die Mischung klar wird, umfasst.

## Revendications

1. Complexe d'inclusion en solution aqueuse comprenant un composé de formule I : 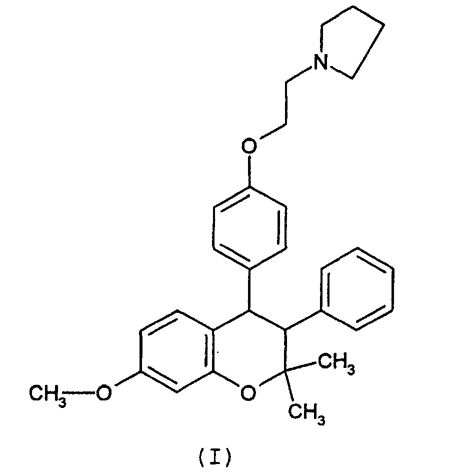 ou un de ses sels acceptables en pharmacie, et une cyclodextrine soluble dans l'eau.

2. Complexe d'inclusion en solution aqueuse selon la revendication 1, dans lequel le composé de formule I est le (-)-3R,4R-trans-7-méthoxy-2,2-diméthyl-3-phényl-4-{4-[2-(pyrrolidin-1-yl)éthoxy]phényl}chromane ou un de ses sels.

3. Complexe d'inclusion selon l'une quelconque des revendications précédentes, dans lequel ledit sel de celui-ci est un sel hydrogénofumarate.

4. Complexe d'inclusion selon l'une quelconque des revendications précédentes, dans lequel la concentration d'un composé de formule I est d'environ 0,1 mg/ml à environ 10 mg/ml, mieux encore d'environ 0,5 mg/ml à environ 5 mg/ml.

5. Complexe d'inclusion selon l'une quelconque des revendications précédentes, dans lequel la concentration d'une cyclodextrine est supérieure à 0,5 %, de préférence d'environ 1,5 % à environ 10 % et mieux encore d'environ 2 % à environ 7 %.

6. Complexe d'inclusion selon l'une quelconque des revendications précédentes, dans lequel ladite cyclodextrine soluble dans l'eau est choisie parmi les hydroxyalkyl-β-cyclodextrines, telles que l'hydroxypropyl-β-cyclodextrine.

7. Formulation pharmaceutique comprenant, à titre d'ingrédient actif, un complexe d'inclusion tel que revendiqué dans l'une quelconque des revendications 1 à 6, en association avec un ou plusieurs véhicules, excipients ou diluants pour celui-ci acceptables en pharmacie.

8. Complexe d'inclusion selon l'une quelconque des revendications 1 à 6, à utiliser en tant qu'agent inhibant la perte osseuse.

9. Utilisation d'un complexe d'inclusion tel que revendiqué dans l'une quelconque des revendications 1 à 6, pour la fabrication d'une formulation pharmaceutique destinée à inhiber la perte osseuse.

10. Procédé pour préparer un complexe d'inclusion selon l'une quelconque des revendications 1 à 6, qui comprend l'addition d'une quantité appropriée d'une cyclodextrine soluble dans l'eau et d'un composé de formule I ou d'un de ses sels, à de l'eau, et l'agitation du mélange jusqu'à ce que le mélange devienne limpide.
